# EUROPÄISCHE PATENTSCHRIFT

(11) **EP 2 480 884 B1**
(45) Veröffentlichungstag und Bekanntmachung des Hinweises auf die Patenterteilung: **08.08.2018**
(21) Anmeldenummer: 10752532.1
(22) Anmeldetag: 11.09.2010
(51) Int. Cl.: G01N 33/487, G01N 35/00

(54) **TESTSTREIFENSTAPEL UND VERFAHREN ZU SEINER HERSTELLUNG**
TEST STRIP STACK AND METHOD FOR ITS PRODUCTION
PILE DE BANDES DE TEST ET SON PROCÉDÉ DE FABRICATION

(30) Priorität: 24.09.2009 EP 09012125
(43) Veröffentlichungstag der Anmeldung: 01.08.2012
(73) Patentinhaber: Roche Diabetes Care GmbH, 68305 Mannheim (DE); F. Hoffmann-La Roche AG, 4070 Basel (CH)
(72) Erfinder: HARTTIG, Herbert, 68434 Neustadt (DE)
(74) Vertreter: Twelmeier Mommer & Partner
(86) Internationale Anmeldenummer: PCT/EP2010/005579
(87) Internationale Veröffentlichungsnummer: WO 2011/035861

(56) Entgegenhaltungen:
- EP-A1- 1 329 395
- EP-A2- 0 547 709
- WO-A1-02/08750
- WO-A2-2007/085438
- US-B1- 6 378 702

## Beschreibung

Die Erfindung betrifft einen Teststreifenstapel mit einzelnen Teststreifen, die übereinander gestapelt sind und ein Testfeld zum Untersuchen einer Körperflüssigkeitsprobe tragen. Ein derartiger Teststreifenstapel ist aus der WO 2007/085438 sowie aus der EP 1 329 395 A1 bekannt. WO0208750 offenbart ein Teststreifenstapel mit einzelnen Teststreifen, die übereinander gestapelt sind und ein Testfeld zur Untersuchung einer Körperflüssigkeitsprobe tragen. Die Teststreifen können einzeln von dem Stapel abgenommen werden. Eine Hochrelieffarbe, die das gesamte Testfeld umgeben kann, wird auf den Teststreifen aufgedruckt. Die Hochrelieffarbe hat im getrockneten Zustand eine Dicke, die größer ist als die Gesamtdicke des Teststreifens in dem Testfeld, wobei die Testfelder in Kammern verpackt sind und benachbarte Teststreifen Boden und Decke einer solchen Kammer bilden. Aus der US 6 378 702 B1 ist ein Teststreifenstapel bekannt, dessen Teststreifen lösbar miteinander verklebt sind, so dass sie einzeln von dem Stapel abgenommen werden können.

Teststreifen ermöglichen zusammen mit entsprechenden Messgeräten eine einfache und schnelle Bestimmung einer Analytkonzentration einer Körperflüssigkeitsprobe, die auch von medizinischen Laien durchgeführt werden kann, beispielsweise zur Messung der Lacatat- oder Cholesterinkonzentration. Teststreifen werden insbesondere auch von Diabetikern verwendet, die mehrmals täglich durch eine Messung der Glucosekonzentration einer Körperflüssigkeitsprobe, in der Regel Blut oder interstitielle Flüssigkeit, ihren Blutzuckerspiegel überprüfen müssen.

Die Testfelder von Teststreifen sind in der Regel empfindlich und müssen deshalb bis zum Gebrauch vor schädlichen Umwelteinflüssen geschützt werden. Aufgabe der vorliegenden Erfindung ist es, hierfür einen kostengünstigen Weg aufzuzeigen.

Diese Aufgabe wird erfindungsgemäß durch einen Teststreifenstapel nach Anspruch 1 gelöst. Die Testfelder der Teststreifen sind in Kammern eingeschlossen, wobei benachbarte Teststreifen Boden und Verschluss einer solchen Kammer bilden. Die Kammern können durch das Verkleben der Streifen gebildet sein, so dass benachbarte Streifen Boden und Decke einer zwischen ihnen liegenden Kammer bilden. Möglich ist es aber auch, dass ein Teststreifen eine Kammer enthält, die eine durch einen benachbarten Streifen verschlossene Öffnung aufweist.

Vorteilhaft ist in einem erfindungsgemäßen Teststreifenstapel jedes einzelne Testfeld bis zu seinem Gebrauch in einer Kammer vor schädlichen Umwelteinflüssen geschützt. Da diese Kammer von den die Testfelder tragenden Streifen gebildet wird, kann mit geringerem Aufwand eine feuchtigkeitsdichte Verpackung erreicht werden.

Bei einem erfindungsgemäßen Verfahren zum Herstellen eines solchen Teststreifenstapels werden einzelne Teststreifen, die auf ihrer Vorderseite ein Testfeld tragen, miteinander zu einem Stapel verklebt, indem jeweils die Vorderseite eines Teststreifens mit der Rückseite eines anderen Teststreifens verklebt wird. Dabei werden die Testfelder in Kammern eingeschlossen und darin feuchtigkeitsdicht versiegelt. Die Testfelder können durch das Verkleben der Streifen in Kammern geschlossen werden, indem benachbarte Streifen Boden und Verschluss einer Kammer bilden. Der Verschluss kann dabei ein Decke einer Kammer bilden oder lediglich eine Öffnung in einer Kammerdecke abdecken.

Zum Verkleben der einzelnen Teststreifen kann auf jeden Teststreifen umlaufend ein Klebstoffstrang aufgebracht werden, so dass die Teststreifen beim Aufeinandersetzen miteinander verkleben. Der Aufwand, auf jeden einzelnen Teststreifen einen separaten Klebstoffstrang aufzutragen, lässt sich vermeiden, indem die einzelnen Teststreifen zum Verkleben in eine Halterung eingelegt werden, welche die einzelnen Streifen in einem Abstand voneinander so hält, dass die Vorderseite eines Streifens der Rückseite des mit ihm zu verklebenden Streifens zugewandt ist. Anschließend kann Klebstoff in die Abstände zwischen den Streifen eingefüllt werden. Vorteilhaft kann auf diese Weise zwischen alle Streifen eines Stapels gleichzeitig Klebstoff eingefüllt werden.

Beispielsweise kann die mittels der Halterung gebildete Teststreifenanordnung mit ihren Seitenflächen in flüssigen Klebstoff getaucht werden, so dass der Klebstoff in die Zwischenräume zwischen den einzelnen Streifen eindringt. Durch die Tiefe, mit welcher die Teststreifenanordnung dabei in dem flüssigen Klebstoff eintaucht, kann vorgegeben werden, auf welcher Breite die Teststreifen an ihren Rändern mit Klebstoff bedeckt werden. Indem zunächst eine Seitenfläche der Teststreifenanordnung in Klebstoff getaucht wird, kann die Anordnung stabilisiert werden, so dass die Halterung entfernt werden kann. Anschließend können die anderen Seitenflächen der Teststreifenanordnung bzw. des Teststreifenstapels in Klebstoff getaucht werden, so dass die Testfelder umlaufend von Klebstoff umgeben sind.

Ein Teststreifenstapel enthält notwendigerweise stets einen Streifen, dessen Vorderseite nicht von einem weiteren Streifen bedeckt ist, da dieser Teststreifen eine Stirnseite des Stapels bildet. Dieser unbedeckte Streifen kann ebenso wie die anderen Streifen des Stapels ein Testfeld tragen. Da ein solches Testfeld jedoch nicht vor schädlichen Umwelteinflüssen geschützt wäre, kann es im Regelfall nicht verwendet werden. Bevorzugt wird der Teststreifenstapel deshalb von einem Streifen ohne Testfeld abgeschlossen. Dies bedeutet, dass das letzte Testfeld eines Stapels von einem Streifen ohne Testfeld bedeckt ist.

Wie erläutert betrifft die vorliegende Erfindung einen Teststreifenstapel aus einzelnen Teststreifen. Das Wort "einzeln" wird im Rahmen der vorliegenden Erfindung gebraucht, um die Teststreifen eines erfindungsgemäßen Teststreifenstapels von einem kontinuierlichen Band zu unterscheiden, das mehrere Testfelder trägt und zu einem Stapel gefaltet ist. Einzelne Teststreifen haben eine Vorderseite, eine Rückseite und - im Gegensatz zu einem endlosen Band - eine umlaufende Seite, welche die Vorderseite mit der Rückseite verbindet. Da Teststreifen in der Regel aus Folie gefertigt sind und deshalb typischerweise recht dünn sind, können die Seitenflächen entsprechend sehr schmal und im Extremfalls nur eine Kante sein. Bei einem einzelnen Teststreifen ist jedoch stets eine umlaufende Seite vorhanden, die in der Regel eine Schnittfläche ist, an welcher der Streifen bei seiner Herstellung aus einer größeren Folie ausgeschnitten wurde.

Weitere Einzelheiten und Vorteile der Erfindung werden an einem Ausführungsbeispiel unter Bezugnahme auf die beigefügten Zeichnungen erläutert. Es zeigen:
- Figur 1:: ein Ausführungsbeispiel eines von einem erfindungsgemäßen Teststreifenstapel abgenommenen Teststreifens;
- Figur 2:: ein Längsschnitt zu Figur 1;
- Figur 3:: ein Ausführungsbeispiel eines erfindungsgemäßen Teststreifenstapels aus Teststreifen gemäß Figur 1 in einer schematischen Darstellung
- Figur 4: eine weiteres Ausführungsbeispiel eines von einem erfindungsgemäßen Teststreifenstapel abgenommenen Teststreifens;
- Figur 5: eine weitere Ansicht zu Figur 4;
- Figur 6: eine Schnittansicht zu Figur 5 entlang der Schnittlinie AA;
- Figur 7: ein weiteres Ausführungsbeispiel eines von einem erfindungsgemäßen Teststreifenstapel abgenommenen Teststreifens;
- Figur 8: eine schematische Darstellung eines Ausschnitts einer Schnittansicht eines Ausführungsbeispiels eines erfindungsgemäßen Teststreifenstapels.

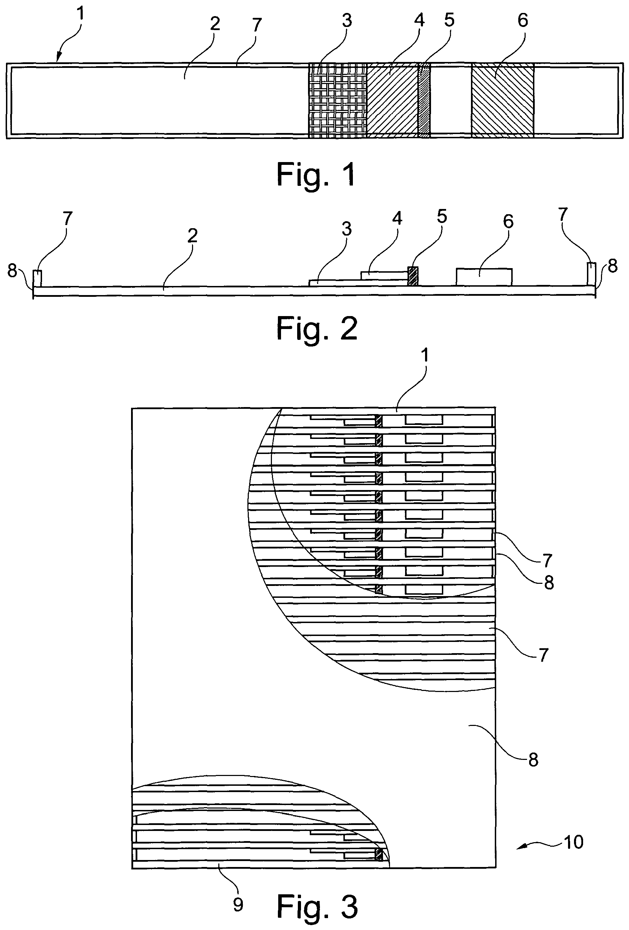

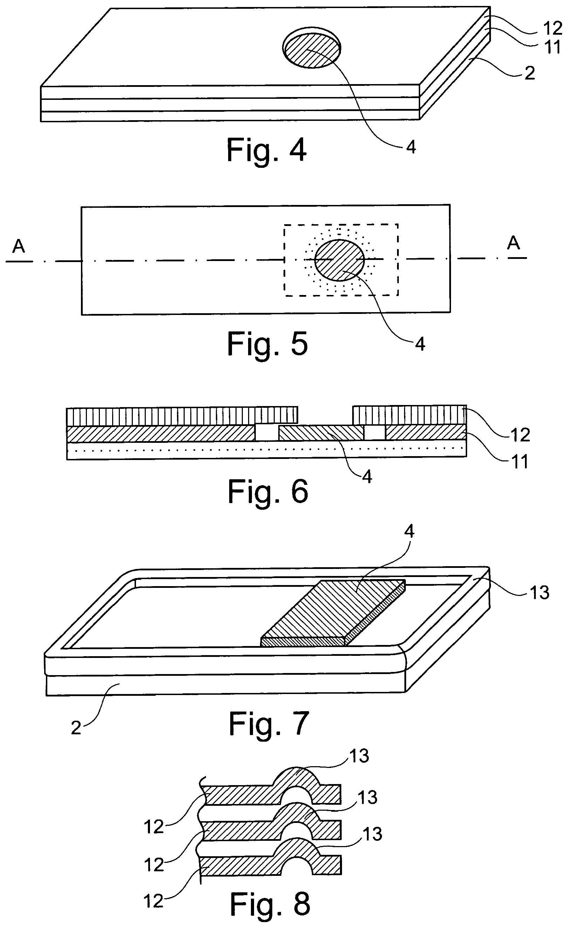

In Figur 1 ist ein Ausführungsbeispiel eines Teststreifens 1 mit Blick auf seine Vorderseite und in Figur 2 in einem Längsschnitt dargestellt. Weitere Ausführungsbeispiele von Teststreifen sind in den Figuren 4 bis 8 dargestellt. Der Teststreifen 1 besteht aus einem streifenförmigen Trägermaterial 2, beispielsweise Kunststofffolie, und trägt auf seiner Vorderseite wenigstens ein, vorzugsweise genau ein, Testfeld 4 zur Untersuchung einer Körperflüssigkeitsprobe. Der Teststreifen 1 kann zusätzlich weitere Hilfsmittel zur Probengewinnung oder Probenuntersuchung tragen, beispielsweise eine Lanzette.

Neben dem Testfeld 4 kann ein Trockenmittel 6 angeordnet sein, beispielsweise auf Basis von Silicagel. Das Trockenmittel 6 kann als Formteil, Pille, Trockenmittelbeutel oder Folie auf dem Trägermaterial 2 des Teststreifens 1 angebracht werden. Möglich ist es auch, das Trockenmittel 6 in Klebstoff 5 zu integrieren, mit dem das Testfeld 4 auf dem Trägermaterial 2 des Streifens festgeklebt ist.

Der Teststreifen 1 weist umlaufend einen Klebstoffstrang 7 auf, der ebenfalls Trockenmittel enthalten kann. Mit dem umlaufenden Klebstoffstrang 7 werden mehrere einzelne Teststreifen 1 zu einem Teststreifenstapel 10 zusammengeklebt, der beispielhaft in Figur 3 mit eine teilweise aufgeschnitten Seitenfläche dargestellt ist.

In dem Teststreifenstapel 10 sind die Testfelder 4 der einzelnen Teststreifen 1 in Kammern angeordnet, die durch das Verkleben der Teststreifen 1 gebildet sind. Dabei bilden benachbarte Streifen 1 Boden und Decke einer zwischen ihnen liegenden Kammer. Seitenwände dieser Kammern werden dabei von dem Klebstoff 7 gebildet, der benachbarte Teststreifen 1 verbindet. Beim Verkleben der Teststreifen 1 werden die Testfelder also in Kammern eingeschlossen. Ein Teststreifen 1, der dabei eine Decke einer solchen Kammer bildet, bildet zugleich auch einen Verschluss der Kammer.

Bevorzugt haben die seitlichen Kammerwände in Stapelrichtung gemessen eine größere Höhe als die in Stapelrichtung gemessene Höhe der Testfelder 4. In diesen Kammern sind die Testfelder 4 bis zum Gebrauch feuchtigkeitsdicht verpackt und vor schädlichen Umwelteinflüssen geschützt.

Der Teststreifenstapel 10 kann an seinen Seitenwänden von einer Schutzschicht 8 umgeben sein, die beispielsweise eine Folie sein kann. Besonders geeignet sind Folien aus Kunststoff und/oder Metall, insbesondere Aluminium. Die Folie 8 ist bevorzugt auf den Stapel 10 aufgeklebt, beispielsweise von dem Klebstoff 7, mit dem auch die einzelnen Teststreifen 1 miteinander verklebt sind. Der die Teststreifen 1 verbindende Klebstoff 7 kann die Seitenwände des Teststreifenstapels 10 bedecken und auf diese Weise ebenfalls eine Schutzschicht bilden. Der Klebstoff bedeckt dabei auch die Seitenflächen der einzelnen Streifen 1.

Eine aus Klebstoff gebildete Schutzschicht kann alternativ oder zusätzlich zu einer die Seitenwände des Stapels 10 bedeckenden Folie 8 verwendet werden. Besonders vorteilhaft kann als Schutzschicht eine Folie 8 verwendet werden, die eine geringe Reißfestigkeit aufweist und deshalb das Abnehmen eines Teststreifens 1 von dem Stapel 10 allenfalls unwesentlich erschwert. Metallfolien, insbesondere Aluminiumfolien, können vorteilhaft sehr dünn ausgebildet sein und eine Weiterreißfestigkeit aufweisen, die geringer als ihre Bruchfestigkeit ist. Auf diese Weise lässt sich erreichen, dass die Folie 8 beim Abnehmen eines Teststreifens 1 von dem Stapel 10 entlang der voneinander zu trennenden Teststreifen 1 reißt.

Die Teststreifen 1 sind durch den Klebstoff 7 lösbar miteinander verklebt, so dass die Teststreifen 1 einzelnen von dem Stapel 10 abgenommen werden können. Geeignete Verklebungen sind beispielsweise zwischen Klebeetiketten und deren Trägerfolien oder Trägerpapieren üblich, von denen die Klebeetiketten zum Gebrauch abgezogen werden können. Als Klebstoff 7 kann beispielsweise auch ein Schmelzkleber verwendet werden.

Um einen einzelnen Teststreifen 1 von dem Stapel 10 abzunehmen, kann auf eine Kante des obersten Teststreifens 1 manuell oder mit einem geeigneten Gerät eine Kraft ausgeübt werden, so dass sich der Teststreifen 1 von dem Stapel 10 löst und abziehen lässt. Um das Abziehen eines Teststreifens 1 zu erleichtern, können die Teststreifen 1 versetzt aufeinander gestapelt sein. Indem ein Teststreifen 1 seitlich oder an einem seiner Enden über den mit ihm verklebten Teststreifen 1 übersteht, lässt sich der abzunehmende Teststreifen leichter fassen und ablösen.

Um zu verhindern, dass versehentlich ein Teilstapel mit zwei oder mehr Teststreifen 1 vom Stapel 10 gelöst wird, kann vor der Krafteinleitung die mechanische Verbindung zwischen dem abzutrennenden Teststreifen 1 und dem restlichen Stapel geschwächt werden, beispielsweise durch Einkerben.

Bevorzugt haftet der Klebstoff 7 an der Vorderseite und der Rückseite der Teststreifen 1 unterschiedlich, so dass er sich beim Abnehmen eines Streifens 1 von dem Stapel 10 stets von derselben Streifenseite löst. Eine unterschiedliche Haftung des Klebstoffs 7 kann beispielsweise durch Aufrauen einer Streifenseite erreicht werden oder indem Vorder- und Rückseite der Streifen 1 aus unterschiedlichem Material bestehen. Besonders vorteilhaft ist es, wenn sich der Klebstoff 1 stets von der Rückseite des Teststreifens 1 löst. Durch eine erhöhte Haftung des Klebstoffs 7 an der Vorderseite des Teststreifens lässt sich vorteilhaft auch das Aufkleben der Testfelder 7 erleichtern, beispielsweise indem die Vorderseite des Streifens 1 eine größere Rauhigkeit als die Rückseite aufweist.

Die Rückseite der Teststreifen 1 kann mit visuell- oder maschinenlesbaren Zeichen, beispielsweise Text, beschrieben sein, beispielsweise mit Informationen zum Abnehmen eines Teststreifens 1 von dem Stapel 10, Hinweisen für deren Verwendung und/oder Produktinformationen. Derartige Informationen können insbesondere aufgedruckt sein.

Die Vorderseite des letzten Streifens eines Teststreifenstapels 10 ist nicht von einem weiteren Streifen bedeckt. Wie bereits erwähnt trägt der letzte Streifen 9 des Stapels 10 bevorzugt kein Testfeld 4. Dieser letzte Streifen 9 kann vorteilhaft als Träger für Produktinformationen, beispielsweise Produktbezeichnung, Lotnummer, Codenummer, Haltbarkeits- und/oder Herstellungsdatum, dienen. Die Produktinformation kann auf den Streifen aufgedruckt sein. Möglich ist es beispielsweise auch, die Produktinformation auf einem separaten Informationsträger, beispielsweise einem Etikett oder einem RFID-Transponder vorzusehen, der auf den Streifen aufgeklebt ist.

Wie bereits erwähnt, kann als Trägermaterial 2 eines Teststreifens 1 eine Kunststofffolie verwendet werden. Gut geeignet sind insbesondere Kunststofffolien mit einer Dicke von 200 bis 400 Mikrometer, beispielsweise 300 Mikrometer. Es können jedoch ohne weiteres auch dickere oder dünnere Kunststofffolien verwendet werden. Die verwendete Folie ist bevorzugt einseitig rau. Geeignete Folien können beispielsweise von der Firma OfoTec Folien GmbH, Nehren, Deutschland unter Bezeichnung "OFOPROP 10775" oder von der Firma DuPont Teijin Films Luxembourg S.A., Luxemburg, unter der Bezeichnung "Melinex" bezogen werden.

Auf die Folie kann mittels einer Klebeschicht ein Streifen eines hydrophilisierten Gewebes fixiert werden. Geeignetes Gewebe wird beispielsweise von der Firma Sefar Holding AG, Thal, Schweiz unter der Bezeichnung "SEFAR PETEX 07-285/44" vertrieben. Auf den Gewebestreifen kann zur Ausbildung eines Testfelds ein Folienstreifen mit einer Nachweisschicht aufgeklebt werden, so dass ein Teil des Gewebes unbedeckt ist. Der Folienstreifen besteht beispielsweise aus Polycarbonat oder einem anderen Kunststoff. Der Folienstreifen kann beispielsweise eine Dicke von 140 Mikrometer haben, wobei auch größere oder kleinere Dicken verwendet werden können. Geeignete Folienstreifen können beispielsweise von der Firma LOFO High Tech GmbH, Weil am Rhein, Deutschland unter der Bezeichnung "Pokalon N 40 GL" bezogen werden. Die von dem Folienstreifen getragene Nachweisschicht enthält Nachweisreagenzien, welche sich unter der Einwirkung von Glukose verfärben. Die Nachweisschicht kontaktiert das hydrophilierte Gewebe.

Die Teststreifen können zusätzlich ein Trockenmittel tragen, beispielsweise eine Folie mit gebundenem Trockenmittel, wie sie von der Firma GRACE GmbH & Co. KG, Worms, Deutschland unter der Bezeichnung "SP566-10414" vertrieben wird. Zusätzlich kann auf die einzelnen Teststreifen eine Lanzette aufgeklebt sein, beispielsweise ein Stechelement aus Flachstahl. Die Vorderseite des Teststreifens kann mit einem Einschnitt versehen sein, der die Vorderseite in zwei Bereiche unterteilt. In einem dieser Bereiche ist das Testfeld, in dem anderen ist das Trockenmittel oder die Lanzette aufgeklebt.

Zur Abdeckung des letzten Testfeldes 4 eines Teststreifenstapels 10 kann ein Streifen 9 aus dem vorstehend beschriebenen Trägermaterial der Teststreifen verwendet werden. Dieser Deckstreifen 9, der bevorzugt kein Testfeld trägt, kann durch einen schwarzen Strich als Abdeckung markiert werden.

Um einen Teststreifenstapel herzustellen, können die einzelnen Teststreifen und der eine Deckstreifen in eine geschlitzte Aufnahme einer Halterung einsortiert werden. Die Anzahl der Teststreifen für einen Teststreifenstapel kann frei gewählt werden. Beispielsweise können 10 bis 100, typischerweise 50 Teststreifen zu einem Stapel verbunden werden.

Nach dem Einsortieren in die Aufnahme werden die Streifen 1 mit einer Seitenfläche, bevorzugt der Längsseite, in eine Kleberschicht gedrückt. Dabei dringt Klebstoff in die Abstände zwischen den einzelnen Streifen 1 ein. Die Klebstoffschicht, in welche der Stapel gedrückt wird, kann vorteilhaft auf einer Folie aufgebracht sein, welche später die Schutzschicht 8 ausbildet. Beispielsweise kann eine Aluminiumfolie, die beispielsweise weniger als 30 Mikrometer, insbesondere weniger als 20 Mikrometer, dick ist, verwendet werden. Geeignet ist insbesondere eine 16 Mikrometer dicke Aluminiumfolie, die mit Schmelzkleber beschichtet wird, beispielsweise mit einem von der Wacker AG, München, Deutschland unter der Bezeichnung "Vinnapas B500/VL20" vertriebenen Schmelzkleber. Der Schmelzkleber wird auf der Folie mit einer Dicke von beispielsweise 100 bis 400 Mikrometern aufgetragen. Bei dem in Figur 3 dargestellten Ausführungsbeispiel wurde eine 200 Mikrometer dicke Schicht verwendet.

Nachdem die Aluminiumfolie auf eine oder zwei Seitenflächen des Stapels aufgeklebt wurde, kann die Halterung entfernt werden, so dass die Aluminiumfolie auch auf die verbleibenden Seitenflächen des Stapels aufgeklebt werden kann. Die Folie ist dabei bevorzugt so bemessen, dass sich ein Überlapp ergibt und die Seitenflächen des Stapels somit lückenlos bedeckt sind.

Auf den Deckstreifen des so gebildeten Stapels kann anschließend ein Selbstklebeetikett mit Produktinformationen aufgeklebt werden.

Mögliche Ausgestaltungen der Teststreifen 1 des Teststreifenstapels 10 sind beispielhaft in den Figuren 1 und 2 sowie 4 bis 8 dargestellt und werden im folgenden erläutert.

Das Testfeld 4 kann, wie bei dem in den Figuren 1 und 2 dargestellten Ausführungsbeispiel, auf einer saugfähigen Schicht 3 angeordnet sein, beispielsweise auf hydrophilem Gewebe oder Vlies. Eine Körperflüssigkeitsprobe kann von der saugfähigen Schicht rasch aufgenommen und in Kontakt mit Nachweisreagenzien des Testfelds 4 gebracht werden, insbesondere wenn die saugfähige Schicht unter dem Testfeld 4 hervorragt und so eine besonders einfache Probenaufgabe ermöglicht.

Das Testfeld 4 kann beispielsweise für eine photometrische oder eine elektrochemische Konzentrationsbestimmung vorgesehen sein. Bevorzugt weist das Testfeld 4 Nachweisreagenzien auf, die bei Kontakt mit einer Körperflüssigkeitsprobe eine Nachweisreaktion bewirken, die zu einer Änderung einer physikalisch messbaren Größe führt, beispielsweise der Farbe oder Farbintensität für einen photometrischen Nachweis bzw. der Leitfähigkeit oder einer anderen elektrischen Größe für einen elektrochemischen Nachweis.

Für eine photometrische Konzentrationsbestimmung kann das Testfeld 4 eine transparente Folie aufweisen, welche die Nachweischemikalien trägt. Bevorzugt wird eine Messung wie bei dem dargestellten Ausführungsbeispiel an der Vorderseite des Teststreifens vorgenommen. Es ist jedoch auch möglich, als Trägermaterial für den Teststreifen eine transparente Folie zu verwenden und dementsprechend die photometrische Messung an der Rückseite des Teststreifens 1 vorzunehmen.

Bei dem in den Figuren 1 und 2 dargestellten Ausführungsbeispiel eines Teststreifens 1 liegt das Testfeld 4 offen auf dem Trägermaterial 2. Das Testfeld 4 bildet auf diese Weise eine Erhebung auf der Vorderseite des Teststreifens 1. In den Figuren 4 bis 6 ist ein Ausführungsbeispiel eines Teststreifens 1 dargestellt, bei dem eine solche Erhebung vermieden werden kann. Der in den Figuren 4 bis 6 dargestellte Teststreifen 1 hat einen sandwichartigen Aufbau.

Bei dem in den Figuren 4 bis 6 dargestellte Teststreifen 1 bildet das Trägermaterial 2 eine Bodenfolie, welche das Testfeld 4 und einen Abstandhalter trägt 11. Als Abstandhalter 11 kann beispielsweise doppelseitig klebendes Klebeband verwendet werden. Der Abstandhalter 11 trägt eine Deckfolie 12, die eine Öffnung aufweist, über welche dem Testfeld 4 eine Körperflüssigkeitsprobe zugeführt werden kann. Bei dem in den Figuren 4 bis 6 dargestellten Ausführungsbeispiel ist die Öffnung über dem Testfeld 4 angeordnet. Es ist aber auch möglich, die Öffnung seitlich versetzt von dem Testfeld 4 anzuordnen und dem Testfeld 4 eine Körperflüssigkeitsprobe von der Öffnung durch einen zwischen der Deckfolie 12 und der Bodenfolie 2 laufenden Kapillarkanal oder ähnliches zuzuführen.

Das Testfeld 4 kann bei dem in den Figuren 4 bis 6 dargestellten Teststreifen 1 für einen elektrochemischen Nachweis ausgebildet sein, beispielsweise indem auf der Bodenfolie 2 oder der Deckfolie 12 Elektroden angebracht sind. Elektroden können beispielsweise aufgedruckt oder durch Laserstrukturieren gebildet sein. Der in den Figuren 4 bis 6 dargestellte Teststreifen 1 kann auch für einen fotometrischen Nachweis ausgebildet sein. Für eine fotometrische Messung kann die Bodenfolie 2 im Bereich des Testfeldes 4 oder sogar vollständig transparent ausgeführt sein. Eine fotometrische Messung kann aber auch von der anderen Seite des Teststreifens 1 durchgeführt werden, beispielsweise durch die Öffnung in der Deckfolie 12 oder, bei einer seitlich versetzt von dem Testfeld 4 angeordneten Öffnung, durch eine zumindest im Bereich des Testfeldes 4 oder sogar vollständig transparent ausgebildete Deckfolie 12 vorgenommen werden.

Der in den Figuren 4 bis 6 dargestellten Teststreifen 1 weist eine durch die Bodenfolie 2, den Abstandshalter 11 und die Deckfolie 12 gebildet Kammer auf. Bei einem Trägerstapel 10 aus derartigen Teststreifen 1 ist diese Kammer durch einen aufgeklebten Teststreifen 1 verschlossen. Die Teststreifen 1 bilden also jeweils einen Verschluss der Kammer eines benachbarten Teststreifens 1.

In den Figuren 7 ist eine weitere Ausführungsform eines Teststreifens 1 dargestellt. Bei diesem Ausführungsbeispiel ist das Testfeld 4 von einem umlaufenden Wulst 13 umgeben, der Teil einer seitlichen Kammerwand ist, welche das Testfeld 4 umschließt. Der Wulst 13 kann beispielsweise durch einen aufgeklebten Rahmen gebildet sein. Figur 8 zeigt schematisch eine weitere Möglichkeit zu Ausbildung eines Wulstes, nämlich durch eine umlaufende Sicke.

### Bezugszahlen

- 1: Teststreifen
- 2: Trägermaterial
- 3: Schicht
- 4: Testfeld
- 5: Klebstoff
- 6: Trockenmittel
- 7: Klebstoff
- 8: Schutzschicht
- 9: Deckstreifen
- 10: Teststreifenstapel
- 11: Abstandshalter
- 12: Deckfolie
- 13: Wulst

## Patentansprüche

1. Teststreifenstapel mit einzelnen Teststreifen (1), die übereinander gestapelt sind und ein Testfeld (4) zur Untersuchung einer Körperflüssigkeitsprobe tragen, wobei aufeinanderliegende Teststreifen (1) lösbar miteinander verklebt sind, so dass die Teststreifen (1) einzeln von dem Stapel (10) abgenommen werden können, wobei die Testfelder (4) in Kammern verpackt sind, benachbarte Teststreifen (1) Boden und Decke einer zwischen ihnen liegenden Kammer bilden, und die Streifen (1) durch zwischen ihnen angeordneten Klebstoff (7) miteinander verklebt sind, der das zwischen den Streifen (1) angeordnete Testfeld (4) umgibt.

2. Stapel nach Anspruch 1, **dadurch gekennzeichnet, dass** die Kammern ein Trockenmittel (6) enthalten.

3. Stapel nach einem der vorstehenden Ansprüche, **dadurch gekennzeichnet, dass** der Klebstoff (7) seitliche Kammerwände bildet.

4. Stapel nach einem der vorstehenden Ansprüche, **dadurch gekennzeichnet, dass** der Klebstoff (7) an der Vorderseite und der Rückseite der Streifen (1) unterschiedlich haftet, so dass er sich beim Abnehmen eines Streifens (1) von dem Stapel (10) stets von derselben Streifenseite löst.

5. Stapel nach einem der vorstehenden Ansprüche, **dadurch gekennzeichnet, dass** der Stapel (10) an seinen Seitenwänden von einer Schutzschicht (8) umgeben ist.

6. Stapel nach einem der vorstehenden Ansprüche, **dadurch gekennzeichnet, dass** die einzelnen Streifen (1) versetzt aufeinander gestapelt sind.

7. Stapel nach einem der vorstehenden Ansprüche, **dadurch gekennzeichnet, dass** der Stapel (10) an seinen Seitenwänden von Klebstoff (7) bedeckt ist.

8. Stapel nach einem der vorstehenden Ansprüche, **dadurch gekennzeichnet, dass** der Stapel (10) von einer Folie (8) umhüllt ist.

9. Stapel nach Anspruch 8, **dadurch gekennzeichnet, dass** die Folie (8) eine Metallfolie ist.

10. Stapel nach einem der vorstehenden Ansprüche, **dadurch gekennzeichnet, dass** die einen Zugang zu dem Testfeld (4) aufweisenden Vorderseiten der Streifen (1) aufgeraut oder mit einem die Haftung des Klebstoffs (7) erhöhendem Material (2) bedeckt sind.

11. Stapel nach einem der vorstehenden Ansprüche, **dadurch gekennzeichnet, dass** die Rückseiten der Streifen (1) mit visuell- oder maschienenlesbaren Zeichen beschrieben sind.

12. Stapel nach einem der vorstehenden Ansprüche, **dadurch gekennzeichnet, dass** das letzte Testfeld (4) eines Stapels (10) von einem Streifen (9) ohne Testfeld (4) bedeckt ist.

13. Verfahren zum Herstellen eines Teststreifenstapels (10) nach einem der vorstehenden Ansprüche, wobei
einzelne Teststreifen (1), die auf ihrer Vorderseite ein Testfeld (4) tragen, miteinander zu einem Stapel (10) verklebt werden, indem jeweils die Vorderseite eines Teststreifens (1) mit der Rückseite eines anderen Teststreifens (10) verklebt wird, und
dabei die Testfelder (4) in Kammern verschlossen werden.

14. Verfahren nach Anspruch 13, **dadurch gekennzeichnet, dass** die einzelnen Teststreifen (1) zum Verkleben in eine Halterung eingelegt werden, welche die einzelnen Streifen (1) in einem Abstand voneinander hält, so dass die Vorderseite eines Streifens (1) der Rückseite des mit ihm zu verklebenden Streifens (1) zugewandt ist, und dann Klebstoff (5) in die Abstände zwischen den Streifen (1) eingefüllt wird.

## Claims

1. A stack of test strips, comprising individual test strips (1) which are stacked on top of each other and carry a test field (4) for examining a body fluid sample, wherein test strips (1) lying on top of one another are detachably adhesively bonded to each other so that test strips (1) can be individually removed from the stack (10), wherein the test fields (4) are packed in chambers and adjacent test strips (1) form the bottom and the closure of such a chamber, and the strips (1) are adhesively bonded to each other by means of an adhesive (7) that is arranged between them and surrounds the test field (4) arranged between the strips (1).

2. The stack according to claim 1, **characterized in that** the chambers contain a desiccant (6).

3. The stack according to any one of the preceding claims, **characterized in** the adhesive (7) forms lateral chamber walls.

4. The stack according to any one of the preceding claims, **characterized in that** the adhesive (7) adheres differently on the front side than on the back side of the strip (1) so that when removing a strip (1) from the stack (10), the adhesive disengages always from the same strip side.

5. The stack according to any one of the preceding claims, **characterized in that** the stack (10) is surrounded at its lateral sides by a protective layer (8).

6. The stack according to any one of the preceding claims, **characterized in that** the individual strips (1) are stacked offset on top of each other.

7. The stack according to any one of the preceding claims, **characterized in that** the stack (10) is covered on its lateral sides with an adhesive (7).

8. The stack according to any one of the preceding claims, **characterized in that** the stack (10) is encased by a film (8).

9. The stack according to claim 8, **characterized in that** the film (8) is a metal film.

10. The stack according to any one of the preceding claims, **characterized in that** the strips' (1) front sides having an access to the test field (4) are roughened or are covered with a material (2) increasing the adhesion of the adhesive (7).

11. The stack according to any one of the preceding claims, **characterized in that** the back sides of the strips (1) are labeled with human- or machine-readable characters.

12. The stack according to any one of the preceding claims, **characterized in that** the last test field (4) of a stack (10) is covered by a strip (9) without test field (4).

13. A method for producing a stack (10) of test strips according to any one of the preceding claims, wherein individual test strips (1) which carry a test field (4) on their front side are adhesively bonded to each other to form a stack (10) in that in each case the front side of a test strip (1) is adhesively bonded to the back side of another test strip (1), and in the course of this, the test fields (4) are enclosed in chambers.

14. The method according to claim 13, **characterized in that** for adhesive bonding, the individual test strips (1) are placed into a holder which holds the individual strips (1) spaced apart from each other so that the front side of a strip (1) faces toward the back side of the strip (1) to which it is to be adhesively bonded, and then adhesive (5) is filled into the gaps between the strips (1).

## Revendications

1. Pile de bandelettes réactives comprenant des bandelettes réactives (1) individuelles qui sont empilées l'une sur l'autre et qui comportent une zone réactive (4) pour analyser un prélèvement de liquide corporel, dans laquelle
des bandelettes réactives (1) superposées sont assemblées l'une à l'autre par collage de façon détachable de telle sorte que les bandelettes réactives (1) peuvent être enlevées individuellement de la pile (10), les zones réactives (4) étant emballées dans des chambres, des bandelettes réactives (1) adjacentes formant le fond et l'élément de recouvrement d'une chambre se trouvant entre deux, et les bandelettes (1) sont assemblées par collage l'une à l'autre par de la colle (7) disposée entre elles, qui entoure la zone réactive (4) disposée entre les bandelettes (1).

2. Pile selon la revendication 1, **caractérisée en ce que** les chambres contiennent un agent déshydratant (6).

3. Pile selon l'une quelconque des revendications précédentes, **caractérisée en ce que** la colle (7) forme des parois latérales de chambre.

4. Pile selon l'une quelconque des revendications précédentes, **caractérisée en ce que** la colle (7) adhère de façon différente sur la face frontale et la face arrière des bandelettes (1), de telle sorte qu'elle se détache lors de l'enlèvement d'une bandelette (1) de la pile (10) invariablement de la même face des bandelettes.

5. Pile selon l'une quelconque des revendications précédentes, **caractérisée en ce que** la pile (10) est entourée par une couche protectrice (8) sur ses parois latérales.

6. Pile selon l'une quelconque des revendications précédentes, **caractérisée en ce que** les bandelettes individuelles (1) sont empilées de façon décalée l'une sur l'autre.

7. Pile selon l'une quelconque des revendications précédentes, **caractérisée en ce que** la pile est recouverte par de la colle (7) sur ses parois latérales.

8. Pile selon l'une quelconque des revendications précédentes, **caractérisée en ce que** la pile (10) est enveloppée par une feuille (8).

9. Pile selon la revendication 8, **caractérisée en ce que** la feuille (8) est une feuille métallique.

10. Pile selon l'une quelconque des revendications précédentes, **caractérisée en ce que** les faces frontales comportant un accès à la zone réactive (4) sont rendues rugueuses ou sont recouvertes d'un matériau (2) augmentant l'adhésion de la colle (7).

11. Pile selon l'une quelconque des revendications précédentes, **caractérisée en ce que** les faces arrière des bandelettes (1) sont marquées avec des signes lisibles visuellement ou par une machine.

12. Pile selon l'une quelconque des revendications précédentes, **caractérisée en ce que** la dernière zone réactive (4) d'une pile (10) est recouverte par une bandelette (9) ne comportant pas de zone réactive (4).

13. Procédé de fabrication d'une pile de bandelettes réactives (10) selon l'une quelconque des revendications précédentes, selon lequel :
des bandelettes réactives (1) individuelles comportant sur leur face frontale une zone réactive (4), sont assemblées par collage l'une à l'autre pour former une pile (10), en collant à chaque fois la face frontale d'une bandelette réactive (1) à la face arrière d'une autre bandelette réactive (1), et
de cette manière les zones réactives (4) sont enfermées dans des chambres.

14. Procédé selon la revendication 13, **caractérisé en ce que** les différentes bandelettes réactives (1) à coller sont insérées dans un support, qui maintient les différentes bandelettes (1) à un certain espacement l'une de l'autre de manière à ce que la face frontale d'une bandelette (1) soit orientée vers la face arrière de la bandelette à coller à celle-ci, et ensuite de la colle (5) est introduite dans les espacements entre les bandelettes (1).
